# EUROPÄISCHE PATENTSCHRIFT

(11) **EP 1 904 433 B1**
(45) Veröffentlichungstag und Bekanntmachung des Hinweises auf die Patenterteilung: **06.10.2010**
(21) Anmeldenummer: 06762416.3
(22) Anmeldetag: 05.07.2006
(51) Int. Cl.: C07C 209/56, C07C 269/00

(54) **VERFAHREN ZUR HERSTELLUNG VON AMINEN AUS HYDROXAMSÄUREN**
METHOD FOR PRODUCING AMINES FROM HYDROXAMIC ACIDS
PROCÉDÉ DE PRODUCTION D'AMINES À PARTIR D'ACIDES HYDROXAMIQUES

(30) Priorität: 11.07.2005 DE 102005032592
(43) Veröffentlichungstag der Anmeldung: 02.04.2008
(73) Patentinhaber: Archimica GmbH, 65929 Frankfurt am Main (DE)
(72) Erfinder: MEUDT, Andreas, 65719 Hofheim (DE); BÖHM, Claudius, 60594 Frankfurt am Main (DE)
(74) Vertreter: Schweitzer, Klaus
(86) Internationale Anmeldenummer: PCT/EP2006/006549
(87) Internationale Veröffentlichungsnummer: WO 2007/006465

(56) Entgegenhaltungen:
- DE-A1- 19 634 446
- US-A- 5 354 891
- TOSHIO ISOBE; TSUTOMU ISHIKAWA: "2-Chloro-1,3-dimethylimidazolinium Chloride. 3. Utility for Chlorination, Oxidation, Reduction, and Rearrangement Reactions" J. ORG. CHEM., Bd. 64, Nr. 16, 1999, Seiten 5832-5835, XP002402651
- "Polyphosphoric acid as a reagent in organic chemistry. IV. Conversion of aromatic acids and their derivatives to amines" JOURNAL OF THE AMERICAN CHEMICAL SOCIETY, Bd. 75, Nr. 8, 1953, Seiten 2014-2015, XP002402652

## Beschreibung

Amine sind bedeutsame und äußerst vielseitig verwendbare Zwischenprodukte in der organischen Synthese. Die Bedeutung in der modernen organischen Synthese wird nur durch Limitierungen der Zugänglichkeit dieser Verbindungsklassen eingeschränkt. Viel häufiger noch als Amine sind die entsprechenden Carbonsäuren oder deren Derivate verfügbar, beispielsweise als Naturstoffe, daher sind alle Methoden zum Abbau von Carbonsäurederivaten zu den entsprechenden Aminen per se von hohem Interesse. Standardverfahren zur Herstellung von primären Aminen aus Carbonsäuren oder Carbonsäurederivaten sind die Hofmann-, Curtius-, Schmidt- und Lossen-Abbaureaktionen.

In der modernen organischen Synthese nimmt die Bedeutung chemo-, regio- und stereoselektiver Reagenzien explosionsartig zu. Will man beispielsweise aus einem komplexen Molekül mit zahlreichen funktionellen Gruppen eine bestimmte Carbonsäuregruppe in ein primäres Amin überführen, so scheiden zahlreiche der genannten Methoden aus Selektivitätsgründen aus. Darüber hinaus stellen Azide, Stickstoffwasserstoffsäure, Brom oder Chlor ein erhebliches Sicherheitsrisiko dar.

Eine hochselektive Problemlösung für die Überführung von Carbonsäuren in primäre Amine, die auch in komplexen multifunktionellen Molekülen mit oft mehr als einem Stereozentrum anwendbar ist, fehlte bisher. Die bekannten Reagenzien können zwar die gewünschten Transformationen bewerkstelligen, dabei werden aber oft andere Gruppierungen ebenfalls beeinflusst. In vielen Fällen werden durch die drastischen erforderlichen Bedingungen selbst entfernt stehende Stereozentren epimerisiert. Zusätzlich sollte die Transformation bei sehr milden Bedingungen anwendbar, die Isolierung des Produktes sehr einfach und die Abtrennung der Folgeprodukte des eingesetzten Reagenzes ohne zusätzliche verfahrenstechnische Schritte möglich sein.

Es wäre daher sehr wünschenswert, ein Verfahren zu haben, das Carbonsäuren durch Umlagerung in die entsprechenden primären Amine überführen kann, dabei aber gleichzeitig sehr milde Reaktionsbedingungen und eine möglichst einfache Aufarbeitung aufweist, und zusätzlich in wirtschaftlich nutzbaren Verfahren einsetzbar ist. Die bekannten Reagenzien lösen dieses Problem nicht, wie an einigen Beispielen demonstriert werden soll: Beim Hofmann-Abbau werden Carbonsäureamide durch Behandlung mit korrosivem und toxischem Brom oder Chlor und Alkali zum primären Amin abgebaut, wobei zahlreiche funktionelle Gruppen aufgrund der hohen Reaktivität der eingesetzten Halogene nicht toleriert werden. Beim Curtius-Abbau kommen giftiges Hydrazin oder explosive Azide, beim Schmidt-Abbau giftige Stickstoffwasserstoffsäure zum Einsatz, wobei letztere bei unsachgemäßer Handhabung ebenfalls Explosionen verursachen kann.

T. Isobe et al., J. Org. Chem. 1999, 64, 5832-5835 beschreiben 2-Chlor-1,3-dimethylimidazolinium Chlorid unter anderem als Agens für Umlagerungs-reaktionen.

DE-A-196 34 446 offenbart enzymatische Synthesen optisch aktiver Hydroxamsäuren und ihre Umsetzung zu optisch aktiven primären Aminen durch Lossen-Umlagerung. Die Lossen-Umlagerung verläuft über die O-acylierte Hydroxamsäure.

H.R. Snyder et al., J. Am. Chem. Soc., Vol. 75, No. 8, 1953, 2014-2015 beschreiben Polyphosphorsäure als Agens für die Umsetzung von aromatischen Carbonsäuren zu Arylaminen (Lossen-Umlagerung) unter CO₂-Abspaltung.

US-A-5,354,891 beschreibt die Herstellung von Derivaten von aromatischen Aminen über eine Lossen-Umlagerung unter Abspaltung von CO₂.

Überraschenderweise wurde gefunden, dass durch Reaktion von Hydroxamsäuren mit Alkylphosphonsäureanhydriden, wie z.B. cyclischen 2,4,6-substituierten 1,3,5,2,4,6-trioxatriphosphinanen, gegebenenfalls in Gegenwart von Basen, alle diese Probleme gelöst werden. Diese Kombination ist eine hochselektive Methode zur Überführung von Carbonsäuren in die entsprechenden carbamatgeschützten Amine, wobei gleichzeitig die gewünschte Epimerisierungsfreiheit und maximale Regio- und Stereoselektivität bei zugleich nahezu quantitativen Ausbeuten beobachtet wird.

Die vorliegende Erfindung betrifft somit ein hochselektives Verfahren zur Herstellung von ungeschützten oder carbamatgeschützten Aminen der Formeln (II) und (III),

R¹ - NH₂ (II)

oder

R¹ - NHCO₂R² (III)

durch Umsetzung von
Hydroxamsäuren der Formel (I)

(R¹ CONHOH) (I)

mit
a.) Alkylphosphonsäureanhydriden,
b.) einem Alkohol R²OH und
c.) optional einer Base
bei einer Temperatur im Bereich von -100 bis + 120°C,
wobei die Hydroxamsäure (I) entweder vor der Umsetzung oder während der Umsetzung (in situ) erzeugt wird und
für einen gegebenenfalls substituierten linearen oder verzweigten C₁-C₁₂-Alkylrest, substituierte C₃-C₁₀ Cycloalkyl-, Alkenyl-, Aryl- oder Heteroarylreste steht und R² steht für einen Allyl-, Aryl-, offenkettigen, cyclischen oder verzweigten C₁ bis C₁₂-Alkyl-Rest, gegebenenfalls substituiert mit offenkettigen, cyclischen oder verzweigten C₁ bis C₁₂ Alkyl-Resten.

Die Hydroxamsäuren können entweder als isolierte reine Edukte eingesetzt werden oder durch eine dem Fachmann bekannten Verfahren zur Herstellung von Hydroxamsäuren entweder vor der Umsetzung oder während der Umsetzung (in situ) generiert werden. Dies kann z. B. erfolgen durch Umsetzung von Carbonsäurehalogeniden und Hydroxylamin oder-salzen, durch Reaktion von Carbonsäureestern mit Hydroxylamin oder -salzen, oder durch Umsetzung von freien Carbonsäuren mit Hydroxylamin oder -salzen in Gegenwart von wasserentziehenden Mitteln. Aufgrund der beschränkten kommerziellen Verfügbarkeit von Hydroxamsäuren ist es bevorzugt, diese z.B. durch eines der oben genannten Verfahren zu generieren und anschließend umgehend in der erhaltenen Reaktionsmischung erfindungsgemäß umzulagern. Insbesondere ist es möglich, ausgehend von Carbonsäuren und Hydroxylamin durch ein weiteres Äquivalent an Alkylphosphonsäureanhydrid die Hydroxamsäure zu bilden und anschließend durch das gleiche Reagenz umzulagern Bevorzugt werden Alkohole R²OH eingesetzt in denen R² für einen Methyl, Ethyl, n-Propyl, Isopropyl, n-Butyl, 2-Butyl, tert-Butyl, Isobutyl, Pentyl, Hexyl, Phenyl, Benzyl insbesondere einen tert.-Butyl oder Benzyl Substituenten steht.

Die Zugabe des Alkohols erfolgt im Allgemeinen mindestens stöchiometrisch im bezug auf die Ausgangsverbindung, kann aber auch überstöchiometrisch sein, beispielsweise im Verhältnis 1 Hydroxamsäure : 1,2 Alkohol.

In einer bevorzugten erfindungsgemäßen Ausführungsform ist das cyclische Phosphonsäureanhydrid ein 2,4,6-substituiertes 1,3,5,2,4,6-trioxatriphosphinan der Formel (IV), 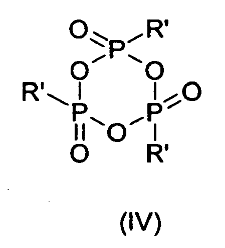 worin R' unabhängig voneinander für Allyl, Aryl oder offenkettige oder verzweigte C₁ bis C₁₂-Alkyl-Reste, insbesondere C₁-C₈-Alkylreste steht.

Bevorzugt werden Phosphonsäureanhydride der Formel (IV) in denen R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht, verwendet.

Die Umlagerung zu ungeschützten Aminen (II) oder carbamatgeschützten Aminen (III) kann im allgemeinen bei Temperaturen im Bereich von -100 bis +120°C durchgeführt werden, bevorzugt sind Temperaturen im Bereich von -30 bis +30°C, wobei tiefere Temperaturen im allgemeinen mit höheren Selektivitäten korreliert sind. Die Reaktionsdauer ist abhängig von der angewandten Temperatur und beträgt im allgemeinen 1 bis 12 Stunden, insbesondere 3 bis 6 Stunden.

Der Zusatz von Basen, insbesondere Aminen, ist im Allgemeinen nicht erforderlich, kann sich aber im Einzelfall als vorteilhaft erweisen. Die Zugabe der Base erfolgt im Allgemeinen mindestens stöchiometrisch im Bezug auf die Ausgangsverbindung, kann aber auch überstöchiometrisch sein, beispielsweise im Verhältnis 1 Hydroxamsäure : 2 Base.

Bevorzugt werden Amine NR³₃ als Base eingesetzt, wobei R³ für einen Allyl-, Aryl-, offenkettigen, cyclischen oder verzweigten C₁ bis C₁₂-Alkyl-Rest, einen Aryloxy-, Allyloxy- oder Alkoxyrest gegebenenfalls substituiert mit offenkettigen, cyclischen oder verzweigten C₁ bis C₁₂-Alkyl-Resten steht und R³ in NR³₃ für verschiedene Reste stehen kann.

Insbesondere steht R³ für H, Methyl, Ethyl, n-Propyl, Isopropyl, n-Butyl, 2-Butyl, Isobutyl, Pentyl, Hexyl, Phenyl, bevorzugt für H, Ethyl, n-Propyl, Isopropyl, n-Butyl, 2-Butyl, Isobutyl oder Phenyl oder eine Kombination der genannten Substituenten.

Das Phosphonsäureanhydrid kann dem Reaktionsmedium entweder als Schmelze oder als flüssige Mischung gelöst in einem Lösungsmittel zugegeben werden. Geeignete Lösungsmitteln sind dabei solche, die keine Nebenreaktionen mit dem Phosphonsäureanhydrid ergeben, dies sind alle aprotischen organischen Lösungsmittel, wie z.B. Ligroin, Butan, Pentan, Hexan, Heptan, Octan, Cyclobutan, Cyclopentan, Cyclohexan, Cycloheptan, Cyclooctan, Dichlormethan, Chloroform, Tetrachlorkohlenstoff, 1,2-Dichlorethan, 1,1,2,2-Tetrachlorethan, Methylacetat, Ethylacetat, Propylacetat, Butylacetat, Dimethylformamid, Diethylformamid, Dimethylacetamid, Diethylacetamid, Diethylether, Diisopropylether, tert-Butyl-Methylether, THF, Dioxan, Acetonitril oder Gemische aus diesen, besonders bevorzugt sind Dichlormethan, Chloroform, Ethylacetat, Propylacetat, Butylacetat, Dimethylformamid, Diethylformamid, Dimethylacetamid, Diethylacetamid, Diisopropylether, tert-Butyl-Methylether, THF, Dioxan, Acetonitril oder Gemische aus diesen, ganz besonders bevorzugt werden Dichlormethan, Chloroform, Ethylacetat, Butylacetat, Dimethylacetamid, tert-Butyl-Methylether, THF, Dioxan, Acetonitril oder Gemische aus diesen, insbesondere bevorzugt sind THF, Ethylacetat oder Butylacetat.

Die Zugabe des Phosphonsäureanhydrids erfolgt im Allgemeinen mindestens drittelstöchiometrisch in Bezug auf die Hydroxamsäure bzw. auf die Ausgangsverbindung zur Erzeugung der Hydroxamsäure, bezogen auf P-Äquivalente, kann aber auch überstöchiometrisch sein, beispielsweise im Verhältnis 1 Carbonsäure : 3 Phosphonsäureanhydrid, bevorzugt 1 Carbonsäure : 2 Phosphonsäureanhydrid.

Die Umsetzungen werden bevorzugt so durchgeführt, dass die entsprechende Hydroxamsäure in einem Lösungsmittel bei der entsprechenden Reaktionstemperatur vorgelegt wird. Anschließend wird die Hydroxamsäure durch Zudosieren des Phosphonsäureanhydrids als Schmelze oder Lösung aktiviert, erwärmt und das gewünschte Amin isoliert. Gegebenenfalls wird vor der Erwärmung der Alkohol zugegeben und das gewünschte carbamatgeschützte Amin isoliert.

In einer weiteren bevorzugten Ausführung des erfindungsgemäßen Verfahrens wird der entsprechende Carbonsäureester mit Hydroxylamin oder einem Salz des Hydroxylamins und einer Base in einem Lösungsmittel vorgelegt wird, wodurch zunächst die Hydroxamsäure vorgebildet wird. Nachdem durch Inprozeßkontrolle sichergestellt wurde, dass die Bildung der Hydroxamsäure vollständig ist, wird anschließend die Umlagerung wie oben beschrieben durchgeführt.

Die Isolierung des Reaktionsproduktes erfolgt bevorzugt durch Hydrolyse und einfache Phasentrennung, da die Folgeprodukte der Phosphonsäureanhydride allgemein sehr gut wasserlöslich sind. Je nach Natur des zu isolierenden Produkts können dabei auch Nachextraktionen erforderlich sein. Das gebildete Phosphonsäureanhydrid-Folgeprodukt stört Folgereaktionen in der Regel nicht, so dass auch der direkte Einsatz der erhaltenen Reaktionslösungen oft sehr gute Ergebnisse bringt.

Alle benannten Verfahrensweisen zeichnen sich durch sehr gute Ausbeuten (typisch 90-100 %, insbesondere > 95 %) bei gleichzeitiger Abwesenheit von Nebenreaktionen und Epimerisierungen aus. Die Selektivitäten der erfindungsgemäßen Reaktion liegen im Bereich von 99-100 %, insbesondere > 99,5 %.

Das erfindungsgemäße Verfahren soll durch die nachfolgenden Beispiele erläutert werden, ohne die Erfindung darauf zu beschränken:

### Beispiele:

Beispiel 1: Umlagerung von Benzhydroxamsäure zu tert.-Butyloxycarbonylanilin 1 mol Benzhydroxamsäure und 2 mol Triethylamin wurden in 50 mL Ethylacetat vorgelegt und auf 0°C gekühlt. 1,2 mol T3P^{®}-Lösung (T3P^{®-} cyclisches Propanphosphonsäureanhydrid, Clariant GmbH) in Ethylacetat (50 % w/w) wurde unter Beibehaltung der Reaktionstemperatur zudosiert, anschließend wurde weitere drei Stunden bei dieser Temperatur nachgerührt. 1.2 mol tert.-Butanol wurde zugegeben und die Reaktionsmischung eine weitere Stunden bei 60°C gerührt. Zu dieser Zeit zeigte das Reaktions-GC einen Umsatz von 100 % an. Nach Erwärmen auf Raumtemperatur wurden 180 mL Wasser zugeben und die Phasen getrennt. Nach Abkondensieren des Lösungsmittels verblieb das Produkt in einer Ausbeute von 97 %, HPLC-Reinheit 98 % (a/a).

### Beispiel 2: Umlagerung von Benzhydroxamsäure zu Benzyloxycarbonylanilin

1 mol Benzhydroxamsäure und 2 mol Triethylamin wurden in 50 mL Ethylacetat vorgelegt und auf 0°C gekühlt. 1,2 mol T3P^{®}-Lösung in Ethylacetat (50 % w/w) wurde unter Beibehaltung der Reaktionstemperatur zudosiert, anschließend wurde weitere drei Stunden bei dieser Temperatur nachgerührt. 1,2 mol Benzylalkohol wurde zugegeben und die Reaktionsmischung eine weitere Stunde bei 60°C gerührt. Zu dieser Zeit zeigte das Reaktions-GC einen Umsatz von 100 % an. Nach Erwärmen auf Raumtemperatur wurden 180 mL Wasser zugeben und die Phasen getrennt. Nach Abkondensieren des Lösungsmittels verblieb das Produkt in einer Ausbeute von 96 %, HPLC-Reinheit 97 % (a/a).

### Beispiel 3: Umlagerung von Octylhydroxamsäure zu Benzyloxycarbonylheptylamin

1 mol Octylhydroxamsäure und 2 mol Triethylamin wurden in 50 mL Ethylacetat vorgelegt und auf 0°C gekühlt. 1,2 mol T3P^{®}-Lösung in Ethylacetat (50 % w/w) wurde unter Beibehaltung der Reaktionstemperatur zudosiert, anschließend wurde weitere drei Stunden bei dieser Temperatur nachgerührt. 1.2 mol Benzylalkohol wurde zugegeben und die Reaktionsmischung eine weitere Stunde bei 60°C gerührt. Zu dieser Zeit zeigte das Reaktions-GC einen Umsatz von 100 % an. Nach Erwärmen auf Raumtemperatur wurden 180 mL Wasser zugeben und die Phasen getrennt. Nach Abkondensieren des Lösungsmittels verblieb das Produkt in einer Ausbeute von 96 %, HPLC-Reinheit 97 % (a/a).

### Beispiel 4: Umlagerung von Octylhydroxamsäure zu tert-Butyloxycarbonylheptylamin

1 mol Octylhydroxamsäure und 2 mol Triethylamin wurden in 50 mL Ethylacetat vorgelegt und auf 0°C gekühlt. 1,2 mol T3P-Lösung in Ethylacetat (50 % w/w) wurde unter Beibehaltung der Reaktionstemperatur zudosiert, anschließend wurde weitere drei Stunden bei dieser Temperatur nachgerührt. 1.2 mol tert.-Butanol wurde zugegeben und die Reaktionsmischung eine weitere Stunde bei 60°C gerührt. Zu dieser Zeit zeigte das Reaktions-GC einen Umsatz von 100 % an. Nach Erwärmen auf Raumtemperatur wurden 180 mL Wasser zugeben und die Phasen getrennt. Nach Abkondensieren des Lösungsmittels verblieb das Produkt in einer Ausbeute von 97 %, GC-Reinheit 97 % (a/a).

## Patentansprüche

1. Verfahren zur Herstellung von ungeschützten oder carbamatgeschützten Aminen der Formeln (II) und (III),
R¹ - NH₂ (II)
oder
R¹ - NHCO₂R² (III)
durch Umsetzung von
Hydroxamsäuren der Formel (I)
(R¹ CONHOH) (I)
mit
a.) Alkylphosphonsäureanhydriden,
b.) einem Alkohol R²OH und
c.) optional einer Base,
bei einer Temperatur im Bereich von -100 bis + 120°C,
wobei die Hydroxamsäure (I) entweder vor der Umsetzung oder während der Umsetzung (in situ) erzeugt wird und
R¹ für einen gegebenenfalls substituierten linearen oder verzweigten C₁-C₁₂-Alkylrest, substituierte C₃-C₁₀ Cycloalkyl-, Alkenyl-, Aryl- oder Heteroarylreste steht und
R² steht für einen Allyl-, Aryl-, offenkettigen, cyclischen oder verzweigten C₁ bis C₁₂-Alkyl-Rest, gegebenenfalls substituiert mit offenkettigen, cyclischen oder verzweigten C₁ bis C₁₂-Alkyl-Resten.

2. Verfahren nach Anspruch 1, **dadurch gekennzeichnet, dass** das Phosphonsäureanhydrid ein 2,4,6-substituiertes 1,3,5,2,4,6-trioxatriphosphinan der Formel (IV) ist. 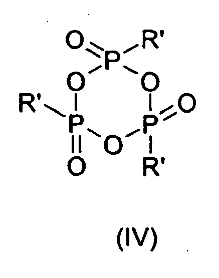 worin R' unabhängig voneinander für Allyl, Aryl oder offenkettige oder verzweigte C₁ bis C₁₂-Alkyl-Reste steht.

3. Verfahren nach Anspruch 2, **dadurch gekennzeichnet, dass** R' für einen Methyl, Ethyl, n-Propyl, Isopropyl, n-Butyl, 2-Butyl, Isobutyl, Pentyl, Hexyl, insbesondere einen Ethyl-, Propyl- und/oder Butyl-Rest steht.

4. Verfahren nach mindestens einem der vorhergehenden Ansprüche, **dadurch gekennzeichnet, dass** das Phosphonsäureanhydrid entweder als Schmelze oder gelöst in einem Lösungsmittel der Reaktionslösung zugegeben wird.

5. Verfahren nach Anspruch 4, **dadurch gekennzeichnet, dass** das Phosphonsäureanhydrid in einem aprotischen Lösungsmittel zugegeben wird.

6. Verfahren nach mindestens einem der vorhergehenden Ansprüche, **dadurch gekennzeichnet, dass** die Reaktionslösung vor Zugabe des Phosphonsäureanhydrids auf die Reaktionstemperatur temperiert wird.

7. Verfahren nach mindestens einem der vorhergehenden Ansprüche, **dadurch gekennzeichnet, dass** das Phosphonsäureanhydrid in Bezug auf die Hydroxamsäure (I) bezogen auf P-Äquivalente drittelstöchiometrisch bis überstöchiometrisch eingesetzt wird.

8. Verfahren nach mindestens einem der vorhergehenden Ansprüche, **dadurch gekennzeichnet, dass** als Base eine Aminbase NR³₃ eingesetzt wird, wobei R³ für
einen Allyl-, Aryl-, offenkettigen, cyclischen oder verzweigten C₁ bis C₁₂-AlkylRest, einen Aryloxy-, Allyloxy- oder Alkoxyrest gegebenenfalls substituiert mit offenkettigen, cyclischen oder verzweigten C₁ bis C₁₂-Alkyl-Resten steht und R³ in NR³₃, für verschiedene Reste stehen kann.

## Claims

1. A method for preparing unprotected or carbamate-protected amines of the formulae (II) and (III),
R¹-NH₂ (II)
or
R¹-NHCO₂R² (III)
by reacting hydroxamic acids of the formula (I)
(R¹CONHOH) (I)
with
a.) alkylphosphonic anhydrides,
b.) an alcohol R²OH and
c.) optionally a base,
at a temperature in the range from -100 to +120 °C,
where the hydroxamic acid (I) is generated either before the reaction or during the reaction (in situ), and
R¹ is an optionally substituted linear or branched C₁-C₁₂-alkyl radical, substituted C₃-C₁₀-cycloalkyl, alkenyl, aryl or heteroaryl radicals, and
R² is an allyl, aryl, open-chain, cyclic or branched C₁ to C₁₂-alkyl radical, optionally substituted by open-chain, cyclic or branched C₁ to C₁₂-alkyl radicals.

2. The method as claimed in claim 1, wherein the phosphonic anhydride is a 2,4,6-substituted 1,3,5,2,4,6-trioxatriphosphinane of the formula (IV) 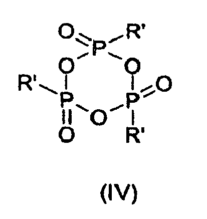 in which R' is independently of one another allyl, aryl or open-chain or branched C₁ to C₁₂-alkyl radicals.

3. The method as claimed in claim 2, wherein R' is a methyl, ethyl, n-propyl, isopropyl, n-butyl, 2-butyl, isobutyl, pentyl, hexyl, especially an ethyl, propyl and/or butyl radical.

4. The method as claimed in at least one of the preceding claims, wherein the phosphonic anhydride is added either as melt or dissolved in a solvent to the reaction solution.

5. The method as claimed in claim 4, wherein the phosphonic anhydride is added in an aprotic solvent.

6. The method as claimed in at least one of the preceding claims, wherein the reaction solution is equilibrated at the reaction temperature before adding the phosphonic anhydride.

7. The method as claimed in at least one of the preceding claims, wherein the amount of the phosphonic anhydride employed is one third stoichiometric to above stoichiometric in relation to the hydroxamic acid (I) based on P equivalents.

8. The method as claimed in at least one of the preceding claims, wherein an amine base NR³₃ is employed as base, where R³ is an allyl, aryl, open-chain, cyclic or branched C₁ to C₁₂-alkyl radical, an aryloxy, allyloxy or alkoxy radical optionally substituted by open-chain, cyclic or branched C₁ to C₁₂-alkyl radicals, and R³ in NR³₃ may represent different radicals.

## Revendications

1. Procédé de production d'amines non protégées ou protégées par un carbamate, de formules (II) et (III),
R¹-NH₂ (II)
ou
R¹-NHCO₂R² (III)
par réaction des acides hydroxamiques de formule (I)
(R¹ CONHOH) (I)
avec
a.) des anhydrides d'acide alkylphosphonique,
b.) un alcool R²OH et
c.) éventuellement, une base,
à une température de l'ordre de -100 à + 120° C
l'acide hydroxamique (I) étant produit soit avant la réaction soit pendant la réaction (in situ) et
R¹ désignant un radical alkyle en C₁ à C₁₂ éventuellement substitué, linéaire ou ramifié, des radicaux cycloalkyle, alcényle, aryle ou hétéroaryle en C₃ à C₁₀ substitués, et
R² désignant un radical allyle, aryle, alkyle en C₁ à C₁₂, à chaîne ouverte, cyclique ou ramifié, éventuellement substitué par des radicaux alkyle en C₁ à C₁₂, à chaîne ouverte, cyclique ou ramifié.

2. Procédé selon la revendication 1, **caractérisé en ce que** l'anhydride d'acide phosphonique est un 1,3,5,2,4,6-trioxatriphosphinane 2,4,6-substitué, de formule (IV), 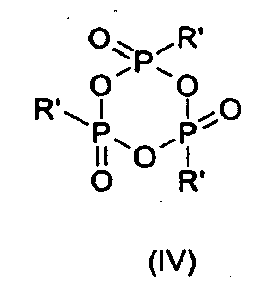 dans laquelle les R' désignent indépendamment les uns des autres, des radicaux allyle, aryle ou alkyle en C₁ à C₁₂ à chaîne ouverte ou ramifié.

3. Procédé selon la revendication 2, **caractérisé en ce que** R' désigne un radical méthyle, éthyle, n-propyle, isopropyle, n-butyle, 2-butyle, isobutyle, pentyle, hexyle, en particulier, un radical éthyle, propyle et/ou butyle.

4. Procédé selon au moins l'une des revendications précédentes, **caractérisé en ce que** l'anhydride d'acide phosphonique est ajouté sous forme de masse fondue ou dissous dans un solvant de la solution réactionnelle.

5. Procédé selon la revendication 4, **caractérisé en ce que** l'anhydride d'acide phosphonique est ajouté dans un solvant aprotique.

6. Procédé selon au moins l'une des revendications précédentes, **caractérisé en ce que** la solution réactionnelle est portée à la température réactionnelle avant d'ajouter l'anhydride d'acide phosphonique.

7. Procédé selon au moins l'une des revendications précédentes, **caractérisé en ce que** l'anhydride d'acide phosphonique est utilisé, en référence à l'acide hydroxamique (I) par rapport aux équivalents P, du tiers stoechiométrique à une façon sur-stoechiométrique.

8. Procédé selon au moins l'une des revendications précédentes, **caractérisé en ce que** l'on utilise comme base, une base d'amine NR³3, dans laquelle R³ désigne
un radical allyle, aryle, alkyle en C₁ à C₁₂ à chaîne ouverte, cyclique ou ramifié, un radical aryloxy, allyloxy ou alcoxy éventuellement substitué par des radicaux alkyle en C₁ à C₁₂ à chaîne ouverte, cyclique ou ramifié et R³ dans NR³3 peut désigner divers radicaux.
